(19) Europäisches Patentamt
European Patent Office
Office européen des brevets

(11) EP 2 172 150 A1

(12) **EUROPEAN PATENT APPLICATION**
published in accordance with Art. 153(4) EPC

(43) Date of publication:
**07.04.2010 Bulletin 2010/14**

(51) Int Cl.:
***A61B 3/12*** *(2006.01)*

(21) Application number: **08764106.4**

(22) Date of filing: **12.06.2008**

(86) International application number:
**PCT/JP2008/001511**

(87) International publication number:
**WO 2009/011088 (22.01.2009 Gazette 2009/04)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **19.07.2007 JP 2007188318**

(71) Applicant: **Kabushiki Kaisha TOPCON**
**Tokyo 174-8580 (JP)**

(72) Inventor: **YUMIKAKE, Kazuhiko**
**Tokyo 174-8580 (JP)**

(74) Representative: **Gagel, Roland**
**Patentanwalt Dr. Roland Gagel**
**Landsberger Strasse 480a**
**81241 München (DE)**

(54) **CORNEA OBSERVATION DEVICE**

(57)    An object is to allow grasp of a depth position of a corneal image. A cornea observation device 100 functions as a full-field OCT device and forms a horizontal tomographic image of a cell of a cornea Ec. A cellular-region extracting part 243 extracts a cellular image region from this tomographic image. A cellular-information generator 244 generates cellular information representing a cellular morphology (size and shape) based on this image region. A memory 247 pre-stores relation information 247a relating a depth position of the cornea to the cellular morphology. A depth-position specifying part 245 selects a depth position corresponding to the cellular morphology of the cornea Ec shown in the cellular information from the relation information 247a and sets as the depth position of the tomographic image. A controller 21 makes a display 22 display this depth position. Consequently, an operator can grasp the depth position of the tomographic image.

## FIG. 2

EP 2 172 150 A1

**Description**

TECHNICAL FIELD

The present invention relates to a cornea observation device for observing the cornea of an eye.

BACKGROUND ART

A slit lamp (a slit lamp biomicroscope), a confocal microscope, a flare cell meter (a flare meter) and so on are known as a device for observing the cornea.
A slit lamp is a device for observing the cross-section of an eyeball by cutting off part of the cornea as an optical section by using a slit light of an illumination light (refer to Patent Document 1, for example). A slit lamp is used not only for observation of each part of the cornea and observation of a lesion but also for observation of a cell such as a corneal endothelial cell.
A confocal microscope is a device for forming an image by detecting, via a pinhole, a reflected light of an illumination light radiated to an eye (refer to Patent Document 2, for example). A confocal microscope is suitable for acquisition of a high-resolution image, and is used for observation of various kinds of cells, collagen fibers and so on of the cornea.
A flare cell meter is a device for measuring the opacity, number of cells, protein concentration and so on of the cornea by detecting a reflected light and scattered light of a laser light radiated to an eye (refer to Patent Document 3, for example).
In recent years, a device using the OCT (Optical Coherence Tomography) technology has drawn attention (refer to Patent Documents 4 and 5, for example). Furthermore, application of such a device (an OCT device) to the ophthalmic field has advanced. An OCT device is a device that superimposes a light propagated through an eye (a signal light) and a light propagated through a reference object (a reference light) to generate an interference light and forms an image based on the detection result of the interference light.
An OCT device enables measurement at high resolution and high sensitivity because using an interferometer. Moreover, an OCT device has an advantage that eye safety is high because using a weak broadband light as an illumination light.
[Patent Document 1] Japanese Unexamined Patent Application Publication No. 9-98950
[Patent Document 2] Japanese Unexamined Patent Application Publication No. 10-90606
[Patent Document 3] Japanese Unexamined Patent Application Publication No. 9-84763
[Patent Document 4] Japanese Unexamined Patent Application Publication No. 2006-153838
[Patent Document 5] Japanese Unexamined Patent Application Publication No. 2006-116028

DISCLOSURE OF THE INVENTION

PROBLEM THAT THE INVENTION IS TO SOLVE

The cornea has a corneal epithelial layer, a Bowman's layer, a corneal stromal layer, a Descemet's membrane and a corneal endothelial layer in order from the corneal surface to the fundus oculi. Moreover, the corneal epithelial layer has superficial cells, wing cells and basal cells in order from the corneal surface to the fundus oculi.
A direction from the corneal surface to the fundus oculi may be referred to as a "depth direction." Moreover, a position in the depth direction may be referred to as a "depth position." Besides, a direction orthogonal to the depth direction may be referred to as a "horizontal direction."
In an observation by an OCT device of the cornea having the structure as described above, it may be required to grasp what depth position in the cornea an image has been acquired. For example, in a follow-up, a preoperative/postoperative observation or the like, there is a need to compare images of (substantially) the same depth positions. For this purpose, there is a need to grasp the depth positions of the respective images. However, it is impossible to grasp the depth position of an image in the cornea by a conventional device.
Further, for early detection of a corneal disease, detection of an abnormality at the cell level is thought to be effective. Since the cornea includes various cells in accordance with the depth positions as described above, the kind of a cell must be specified in order to detect an abnormality. However, with a conventional device, it is impossible to specify the kind of a cell, and therefore, it is impossible to detect whether there is an abnormality in a cell of the cornea.
The present invention was made to solve the above problems, and an object of the present invention is to provide a cornea observation device that allows grasp of the depth position of an image in the cornea.

Another object of the present invention is to provide a cornea observation device that allows determination of the state of an abnormality in a cell of the cornea.

MEANS FOR SOLVING THE PROBLEM

In order to achieve the above objects, in a first aspect of the present invention, a cornea observation device comprises: an image forming part configured to radiate a light to an eye, detect the light propagated though a cornea, and form an image of a cell of the cornea based on a result of the detection; and a specifying part configured to analyze a morphology of the cell shown in the image and specify a depth position of the cornea shown in the image.

In a second aspect of the present invention, the cornea observation device according to the first aspect is **characterized in that** the specifying part is configured to specify a layer of the cornea shown in the image as the depth position.

In a third aspect of the present invention, the cornea observation device according to the first aspect is **characterized in that** the specifying part includes an extracting part configured to extract an image region of at least one cell in the image and a generator configured to generate cellular information on the morphology of the cell of the cornea based on the image region, and is configured to specify the depth position of the image based on the cellular information.

In a fourth aspect of the present invention, the cornea observation device according to the third aspect is **characterized in that** the specifying part includes a determining part configured to determine whether there is an abnormality of the cell of the cornea based on the cellular information.

In a fifth aspect of the present invention, the cornea observation device according to the fourth aspect is **characterized in that**: the image forming part is configured to form a two-dimensional image of the cornea in a cross-section substantially orthogonal to a depth direction of the cornea; the generator is configured to generate the cellular information including morphology information of a cross-section of a cell shown in the two-dimensional image; and the determining part is configured to determine whether there is an abnormality of the cell in the cross-section based on the morphology information.

In a sixth aspect of the present invention, the cornea observation device according to the fourth aspect is **characterized in that**: the image forming part is configured to generate two-dimensional images of the cornea in a plurality of cross-sections substantially orthogonal to a depth direction of the cornea, respectively, and form a tomographic image in a cross-section along the depth direction based on the plurality of two-dimensional images; the generator is configured to generate the cellular information including morphology information of a cross-section of a cell shown in the tomographic image and/or morphology information of a cell layer shown in the tomographic image; and the determining part is configured to determine whether there is an abnormality of a cell in the cross-section along the depth direction based on the morphology information.

In a seventh aspect of the present invention, the cornea observation device according to the fourth aspect is **characterized in that** the determining part is configured to previously store an allowable range of a morphology of a cell of a cornea, determine whether the morphology represented in the cellular information is included in the allowable range, and determine the cell of the cornea is normal when determining the morphology is included, whereas determine the cell of the cornea is abnormal when determining the morphology is not included.

In an eighth aspect of the present invention, the cornea observation device according to the seventh aspect is **characterized in that** the determining part is configured to previously store the allowable range at each of a plurality of depth positions of a cornea, and select the allowable range corresponding to the depth position specified by the specifying part to execute the determination.

In a ninth aspect of the present invention, the cornea observation device according to the fourth aspect further comprises a storing part configured to store the cellular information, and is **characterized in that** the determining part is configured to, when new cellular information is generated by the generator, compare the cellular information previously stored in the storing part with the new cellular information and determine a change of the morphology of the cell of the cornea.

In a tenth aspect of the present invention, the cornea observation device according to the third aspect is **characterized in that** the specifying part includes a memory part configured to previously store relation information that relates a depth position in a cornea to a morphology of a cell, and is configured to select a depth position corresponding to the morphology represented in the cellular information from the relation information and set the selected depth position as the depth position of the image.

In an eleventh aspect of the present invention, the cornea observation device according to the third aspect is **characterized in that** the morphology of the cell includes a size and/or shape of a cell of a cornea.

In a twelfth aspect of the present invention, the cornea observation device according to the third aspect is **characterized in that**: the generator is configured to generate density information that represents a density of the cell of the cornea based on the image region, as the cellular information; and the specifying part is configured to specify the depth position of the image based on the density information.

In a thirteenth aspect of the present invention, the cornea observation device according to the twelfth aspect is **characterized in that** the specifying part includes a memory part configured to previously store relation information that relates a depth position in a cornea to a density of a cell, and is configured to select a depth position corresponding to the density represented in the density information from the relation information and set the selected depth position as the depth position of the image.

In a fourteenth aspect of the present invention, the cornea observation device according to the third aspect is **characterized in that**: the extracting part is configured to extract image regions of a plurality of cells; and the generator is configured to execute a statistical process on the image regions of the plurality of cells and generate the cellular information.

In a fifteenth aspect of the present invention, the cornea observation device according to the first aspect is **characterized in that** the specifying part is configured to analyze the morphology of the cell based on pixel values of pixels composing the image and to specify the depth position of the image.

In a sixteenth aspect of the present invention, the cornea observation device according to the fifteenth aspect is **characterized in that** the specifying part includes a memory part configured to previously store relation information that relates a depth position in a cornea to pixel values of pixels composing an image of the cornea, and is configured to select a depth position corresponding to pixel values of pixels composing the image formed by the image forming part from the relation information and to set the selected depth position as the depth position of the image.

In a seventeenth aspect of the present invention, the cornea observation device according to the fifteenth aspect is **characterized in that** the specifying part is configured to execute a statistical process on pixels values of pixels composing the image and specify the depth position of the image based on a result of the statistical process.

In an eighteenth aspect of the present invention, the cornea observation device according to the fifteenth aspect is characterized in that the specifying part includes a determining part configured to determine whether there is an abnormality of the cell of the cornea based on pixel values of pixels composing the image.

In a nineteenth aspect of the present invention, the cornea observation device according to the fourth aspect is provided with an output part configured to output a result of determination by the determining part and the depth position of the image specified by the specifying part.

In a twentieth aspect of the present invention, the cornea observation device according to the eighteenth aspect is provided with an output part configured to output a result of determination by the determining part and the depth position of the image specified by the specifying part.

In a twenty-first aspect of the present invention, the cornea observation device according to the fourth aspect is **characterized in that** the specifying part includes a measuring part configured to measure a size of a cell determined to have an abnormality by the determining part.

In a twenty-second aspect of the present invention, the cornea observation device according to the eighteenth aspect is **characterized in that** the specifying part includes a measuring part configured to measure a size of a cell determined to have an abnormality by the determining part.

In a twenty-third aspect of the present invention, a cornea observation device, comprising: an image forming part configured to radiate a light to an eye, detect the light propagated though a cornea, and form an image of a cell of the cornea based on a result of the detection; and an analyzer configured to analyze the morphology of the cell shown in the image and to determine whether there is an abnormality of the cell of the cornea.

In a twenty-fourth aspect of the present invention, the cornea observation device according to the first aspect is **characterized in that** the image forming part is an OCT device including an interference-light generator configured to split a broadband light into a signal light and a reference light and superimpose the signal light propagated through a cornea and the reference light propagated through a reference object to generate an interference light, a detector configured to detect the interference light, and a forming part configured to form an image of a cell of the cornea based on a detection result of the interference light.

In a twenty-fifth aspect of the present invention, the cornea observation device according to the twenty-third aspect is **characterized in that** the image forming part is an OCT device including an interference-light generator configured to split a broadband light into a signal light and a reference light and superimpose the signal light propagated through a cornea and the reference light propagated through a reference object to generate an interference light, a detector configured to detect the interference light, and a forming part configured to form an image of a cell of the cornea based on a detection result of the interference light.

In a twenty-sixth aspect of the present invention, the cornea observation device according to the twenty-fourth aspect is **characterized in that**: the interference-light generator is configured to radiate the signal light having a predetermined beam diameter to the cornea and generate the interference light having a predetermined beam diameter; the detector is configured to detect the interference light on a two-dimensional light-receiving face; and the forming part is configured to form a two-dimensional image of a region of the cornea corresponding to the beam diameter of the signal light, as the image.

In a twenty-seventh aspect of the present invention, the cornea observation device according to the twenty-fifth aspect is **characterized in that**: the interference-light generator is configured to radiate the signal light having a predetermined beam diameter to the cornea and generate the interference light having a predetermined beam diameter; the detector is configured to detect the interference light on a two-dimensional light-receiving face; and the forming part is configured to form a two-dimensional image of a region of the cornea corresponding to the beam diameter of the signal light, as the image.

In a twenty-eighth aspect of the present invention, the cornea observation device according to the twenty-fourth aspect is **characterized in that** the image forming part includes a changing part configured to change a difference in optical path length between the signal light and the reference light, and is configured to form an image of the cornea at a depth position corresponding to the difference in optical path length.

In a twenty-ninth aspect of the present invention, the cornea observation device according to the twenty-fifth aspect is **characterized in that** the image forming part includes a changing part configured to change a difference in optical path length between the signal light and the reference light, and is configured to form an image of the cornea at a depth position corresponding to the difference in optical path length.

EFFECT OF THE INVENTION

According to the cornea observation device of the present invention, it is possible to grasp the depth position of an image in the cornea because it is possible to form an image of a cell of the cornea and analyze the morphology of the cell shown in this image to specify the depth position of the image.

Further, according to the cornea observation device of the present invention, it is possible to determine the state of an abnormality in a cell of the cornea because it is possible to extract an image region of the cell from an image of the cell of the cornea, generate cellular information based on this image region and determine whether there is an abnormality in the cell of the cornea based on this cellular information.

Further, according to the cornea observation device of the present invention, it is possible to determine the state of an abnormality in a cell of the cornea because it is possible to form an image of the cell of the cornea and analyze the morphology of the cell shown in this image to determine whether there is an abnormality in the cell of the cornea.

BRIEF DESCRIPTION OF THE DRAWINGS

Fig. 1 is a schematic view showing an example of the entire configuration of an embodiment of a cornea observation device according to the present invention.
Fig. 2 is a schematic block diagram showing an example of the configuration of a control system of the embodiment of the cornea observation device according to the present invention.
Fig. 3 is a flow chart showing an example of an operation pattern of the embodiment of the cornea observation device according to the present invention.

DESCRIPTION OF REFERENCE NUMERALS AND SYMBOLS

| | |
|---|---|
| 100 | cornea observation device |
| 1 | halogen lamp |
| 2 | filter |
| 3 | beam splitter |
| 4 | waveplate |
| 5 | polarization plate |
| 6, 15 | reflection mirrors |
| 7 | glass plate |
| 8, 11 | objective lenses |
| 9 | reference mirror |
| 10 | reference-mirror moving mechanism |
| 12 | aperture diaphragm |
| 13 | imaging lens (lenses) |
| 14 | polarized-beam splitter |
| 16, 17 | CCDs |
| 20 | computer |

| 21 | controller |
| 22 | display |
| 23 | manipulation part |
| 24 | signal processor |
| 241 | image forming part |
| 242 | analyzer |
| 243 | cellular-region extracting part |
| 244 | cellular-information generator |
| 245 | depth-position specifying part |
| 246 | abnormality determining part |
| 247 | memory |
| 247a | relation information |
| 247b | association information |

BEST MODE FOR CARRYING OUT THE INVENTION

An embodiment of a cornea observation device according to the present invention will be described. This cornea observation device is a device for observing a minute structure such as cells and collagen fibers of the cornea. The minute structure of the cornea may be generically referred to as a "cell."

[CONFIGURATION]

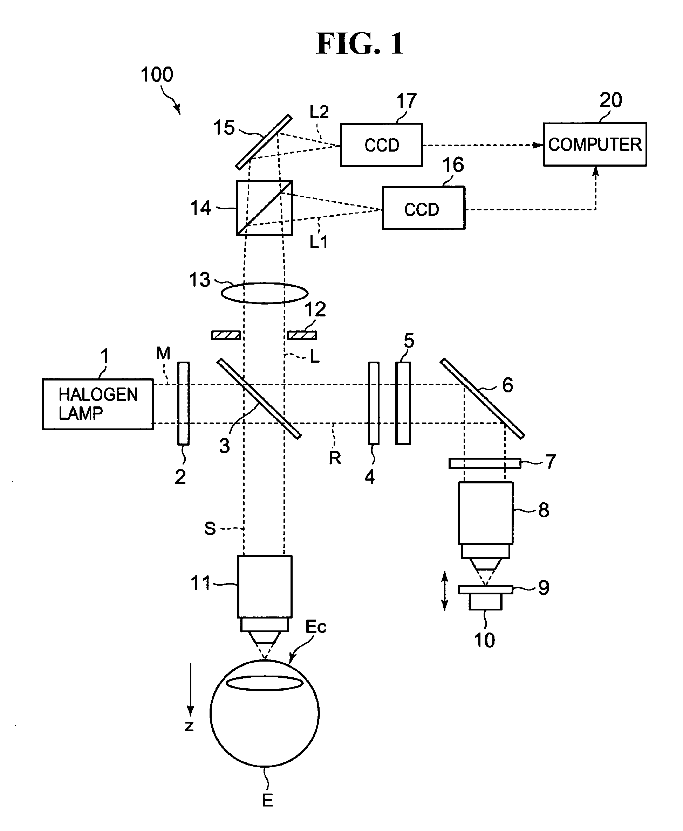

An example of the configuration of the cornea observation device according to this embodiment is shown in Fig. 1. A cornea observation device 100 employs a full-field OCT device as an "image forming part" that forms an image of a cell of the cornea.

A full-field OCT device is a device that, as the OCT device described in Patent Document 4 mentioned before, radiates a signal light having a predetermined beam diameter to the cornea and detects an interference light having a predetermined beam diameter by a two-dimensional optical sensor array, thereby acquiring a two-dimensional image of a corneal region corresponding to the beam diameter of the signal light.

A full-field OCT device has higher resolution than a cornea observation device (a slit lamp, a confocal microscope, a flare cell meter, and the like) other than an OCT device and than other types (Fourier-domain, swept-source, and the like) of OCT devices.

A full-field OCT device is also referred to as an en-face OCT device.

An eye E is placed in a suitable state for measurement. For example, in a case that the eye E is a living eye, jelly or liquid for minimizing change of the refractive index at the boundary can be applied to the eye E. Further, in a case that the eye E is an isolated eye, the eye E can be placed in the immersed condition in order to minimize change of the refractive index at the boundary.

The cornea observation device 100 is provided with a halogen lamp 1 as a light source. The halogen lamp 1 emits, for example, a non-polarized broadband light M. The halogen lamp 1 may include, together with a general halogen lamp, an optical fiber bundle that guides the emitted light, a Kohler illumination optical system for uniformly illuminating the radiation field of the emitted light, and so on, which are not shown in the drawings. The non-polarized broadband light M emitted from the halogen lamp 1 has a predetermined beam diameter.

The light source is not limited to the halogen lamp 1, and may be any light source that emits a non-polarized broadband light. For example, it is possible to employ any thermal light source (a light source using black-body radiation) such as a xenon lamp. Further, the light source may be a laser light source that emits a randomly polarized broadband light. Here, "non-polarized" means a polarization condition including a linearly polarized light, a circularly polarized light and an elliptically polarized light. Further, "randomly polarized" means a polarization condition in which there are two linear-polarization components orthogonal to each other and the power of each of the linear-polarization components varies temporally at random (refer to Japanese Unexamined Patent Application Publication No. 7-92656). Although only the case of non-polarization will be described in detail below, it is also possible in the case of random polarization to obtain similar actions and effects with a similar configuration.

The broadband light M emitted by the halogen lamp 1 includes lights of various bands. A filter 2 is a filter that transmits only a predetermined band of the non-polarized broadband light M. A band to transmit is determined based on the resolution, measurement depth or the like, and set to a band whose central wavelength is about 760 nm and wavelength width is about 100 nm, for example. In this case, it is possible to acquire an image having resolution of about 2 $\mu$m with respect to the depth direction (the z-direction in Fig. 1) of the eye E and the direction orthogonal thereto (the horizontal direction). The light transmitted by the filter 2 will be also referred to as the broadband light M.

The non-polarized broadband light M transmitted by the filter 2 is split into two by a beam splitter 3 such as a half mirror. To be specific, a reflected light by the beam splitter 3 forms a signal light S, and a transmitted light by the beam splitter 3 forms a reference light R.

The signal light S is focused on the eye E by an objective lens 11 while being kept in the non-polarized state. The signal light S is radiated with a predetermined beam diameter to a cornea Ec. A light reflected or scattered on the surface or inside of the eye E returns to the beam splitter 3 through the objective lens 11.

On the other hand, the non-polarized reference light R generated by the beam splitter 3 is transmitted by a waveplate 4 (a λ/4 plate) and a polarization plate 5, and reflected by a reflection mirror 6. Further, the reference light R is transmitted by a glass plate 7, and focused on the reflection face of a reference mirror 9 by an objective lens 8. The reference light R reflected by the reference mirror 9 is reversely propagated on the same optical path to return to the beam splitter 3.

In this process, the reference light R that is initially non-polarized is propagated through the waveplate 4 and the polarization plate 5 twice to be converted into a circularly polarized light. The waveplate 4 and the polarization plate 5 are examples of the "converter" of the present invention. The glass plate 7 is a dispersion correction optical element that minimizes the influence of dispersion occurring on the optical paths of the signal light S and the reference light R (both arms of an interferometer).

The reference mirror 9 is configured to be movable by a reference-mirror moving mechanism 10 in a travelling direction of the reference light R, that is, a direction orthogonal to the reflection face of the reference mirror 9 (a direction of a double-sided arrow sign in Fig. 1). The reference-mirror moving mechanism 10 includes a driver such as a piezo element.

A difference in optical path length between the signal light S and the reference light R is changed by moving the reference mirror 9 in the above manner. The optical path length of the signal light S is an out-and-return distance between the beam splitter 3 and the surface of the cornea Ec. Moreover, the optical path length of the reference light R is an out-and-return distance between the beam splitter 3 and the reflection face of the reference mirror 9. By changing a difference in optical path length between the signal light S and the reference light R, it is possible to selectively acquire images at various depth positions of the cornea Ec. The reference-mirror moving mechanism 10 is an example of the "changing part" of the present invention.

Although the aforementioned difference in optical path length is changed by changing the optical path length of the reference light R in this embodiment, it is also possible to configure to change the aforementioned difference in optical path length by changing the optical path length of the signal light S. In this case, such a changing part that changes an interval between a device optical system and the eye E is installed. For example, it is possible to employ, as the changing part, a stage that moves the device optical system in the z-direction or a stage that moves the eye E in the z-direction.

The signal light S propagated through the eye E and the reference light R propagated through the reference mirror 9 are superimposed by the beam splitter 3, whereby an interference light L is generated. The interference light L includes S-polarization components and P-polarization components. An interferometer including the halogen lamp 1, the beam splitter 3 and the reference mirror 9 is an example of the "interference-light generator" of the present invention.

The interference light L generated by the beam splitter 3 is propagated through an aperture diaphragm 12 and made to become a focused light by an imaging lens (lenses) 13. S-polarization components L1 of the interference light L having become a focused light are reflected by a polarized-beam splitter 14 and detected by a CCD (image sensor) 16. On the other hand, P-polarization components L2 of the interference light L are transmitted by the polarized-beam splitter 14, reflected by a reflection mirror 15, and detected by a CCD (image sensor) 17.

Each of the CCDs 16 and 17 has a two-dimensional light-receiving face. The S-polarization components L1 and the P-polarization components L2 are respectively radiated with predetermined diameters to the light-receiving faces of the CCDs 16 and 17.

The CCDs 16 and 17 having detected the S-polarization components L1 and the P-polarization components L2 transmit detection signals to a computer 20, respectively. The CCDs 16 and 17 are examples of the "detector" of the present invention.

Since the reference light R and the signal light S, which become the interference light L, are circularly polarized and non-polarized, respectively, the S-polarization components L1 and the P-polarization components L2 have a phase difference of 90° ($\pi/2$). Therefore, a detection signal $C_A$ outputted from the CCD 16 and a detection signal $C_B$ outputted from the CCD 17 have a phase difference of 90°, and can be expressed in an equation described below.

[EQUATION 1]

$$C_A(x,y) = I_s(x,y) + I_r(x,y) + \sqrt{I_s(x,y)I_r(x,y)}\cos(\Delta\phi(x,y)) \qquad (1)$$

$$C_B(x,y) = I_s(x,y) + I_r(x,y) + \sqrt{I_s(x,y)I_r(x,y)}\sin(\Delta\phi(x,y)) \qquad (2)$$

In this equation, $I_s(x,y)$ represents the intensity of the signal light S, and $I_r(x,y)$ represents the intensity of the reference light R. Moreover, $\phi(x,y)$ represents an initial phase difference. Moreover, each of the detection signals $C_A$ and $C_B$ includes a backlight component (a noninterference component, a direct current component) $I_s(x,y)+I_r(x,y)$. Further, the detection signal $C_A$ includes an interference component composed of a cos component, and the detection signal $C_B$ includes an interference component composed of a sin component.

As apparent from the equations 1 and 2, each of the detection signals $C_A$ and $C_B$ includes only space (the x-direction and y-direction orthogonal to the z-direction) as a variable, and does not include time as a variable. That is to say, the interference signal in this embodiment includes only a special change.

[Configuration of Control System]

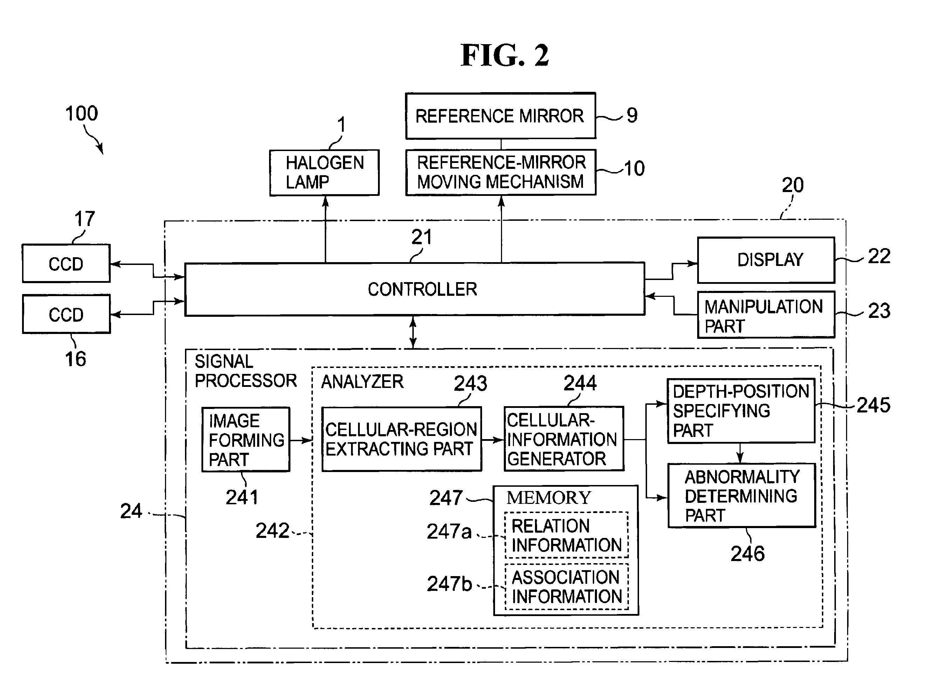

The configuration of a control system of the cornea observation device 100 will be described. Fig. 2 shows an example of the configuration of the control system of the cornea observation device 100.

The computer 20 is provided with a controller 21, a display 22, a manipulation part 23, and a signal processor 24.

(Controller)

The controller 21 controls each part of the cornea observation device 100. For example, the controller 21 executes control of the halogen lamp 1 to turn on/off, control of the reference-mirror moving mechanism 10, control of the exposure times of the CCDs 16 and 17, control of a display process by the display 22, and so on.

The controller 21 includes a microprocessor such as a CPU. Moreover, the controller 21 includes a memory device such as a RAM, a ROM and a hard disk drive. The hard disk drive previously stores a computer program for device control (not shown). Through the operation of the microprocessor in accordance with the computer program, the aforementioned controls by the controller 21 are executed.

Further, the controller 21 may be provided with communication equipment for data communication with an external device. The communication equipment is, for example, a LAN card and a modem. Thus, the controller 21 can acquire various kinds of information from an external database and register information into the database. Moreover, the controller 21 can acquire information from an ophthalmic device such as an examination device and transmit information to the ophthalmic device.

(Display)

The display 22 is controlled by the controller 21 to display various kinds of information. The display 22 includes any display device such as an LCD or a CRT display.

(Manipulation Part)

The manipulation part 23 is used when the operator manipulates the cornea observation device 100 and inputs various kinds of information. The manipulation part 23 includes any manipulation device and input device such as a mouse, a keyboard, a joystick, a trackball and a dedicated control panel.

(Signal Processor)

The signal processor 24 processes various kinds of signals. The signal processor 24 includes a microprocessor such as a CPU, a RAM, a ROM, a hard disk drive, and so on. The signal processor 24 is provided with an image forming part 241 and an analyzer 242.

(Image Forming Part)

The image forming part 241 forms an image of the eye E, specifically, a horizontal image of the cornea Ec, based on the detection signals $C_A$ and $C_B$ outputted from the CCDs 16 and 17. This image forming process will be described later. The image forming part 241 is an example of the "forming part" of the present invention.

(Analyzer)

The analyzer 242 analyzes an image formed by the image forming part 241. To be specific, the analyzer 242 analyzes a horizontal image of the cornea Ec and specifies the depth position of the image in the cornea Ec. The analyzer 242 is an example of the "specifying part" of the present invention.
The analyzer 242 is provided with a cellular-region extracting part 243, a cellular-information generator 244, a depth-position specifying part 245, an abnormality determining part 246, and a memory 247.

(Memory)

The memory 247 previously stores relation information 247a. The memory 247 is an example of the "memory part" of the present invention.
The relation information 247a is information that relates the depth position in the cornea to the morphology of a cell. The morphology of a cell includes the size, shape and so on of the cell. The size of a cell is, for example, the diameter, the peripheral length, and the area. The relation information 247a relates various depth positions of the cornea, namely, various layers composing the cornea to the morphologies of a cell at the respective depth positions (layers).
The depth position in the cornea may be defined by numerical value information (coordinate information) such as a distance from a reference position in the cornea (for example, the corneal surface), or may be defined by a range in the depth direction of the cornea, such as the name of a layer of the cornea or the depth in each layer (for example, the surface layer, middle layer and deep layer of the corneal stromal layer).
The relation information 247a is obtained by, for example, clinically acquiring images at various depth positions in the cornea and generating statistical association between the depth positions in the cornea and the morphologies of cells based on the images.
In a case that an examination has been executed on the eye before, the relation information 247a unique to the eye may be generated based on the result of the examination. In this case, it is possible to store the relation information 247a together with identification information of the eye (or the subject), and read out and use the relation information 247a with the identification information as a search tag when necessary.
As mentioned before, the cornea includes the corneal epithelial layer, the Bowman's layer, the corneal stromal layer, the Descemet's membrane, and the corneal endothelial layer.
The corneal epithelial layer has a thickness of approximately 50 $\mu$m and is composed of five to seven cell layers. The corneal epithelial layer includes superficial cells, wing cells and basal cells. The superficial cells exist in the two to three layers from the corneal surface. The superficial cell is a flat cell having a thickness of approximately 4 $\mu$m and a width of approximately 40 $\mu$m. The superficial cells are firmly joined to each other while the respective layers are alternately arranged (which is called tight junction), thereby protecting the cornea from an influence from outside. The basal cell is a columnar cell having a thickness of approximately 18 $\mu$m and a width of approximately 10 $\mu$m. The wing cell has an intermediate shape between the basal cell and the superficial cell. The basal cells divide. The divided basal cells gradually flatten while going up to the corneal surface. Thus, the cells included in the corneal epithelial layer have characteristic morphologies depending on the depths from the corneal surface.
The Bowman's layer is a layer having a uniform thickness of about 12 $\mu$m, and has a felt-like structure in which collagen fibers are irregularly arranged.
The corneal stroma accounts for approximately 90% of the corneal thickness. The corneal stroma is composed of keratocytes that have capacity to produce components of the extracellular matrix including layer plates of collagen fibers formed by approximately 200 layers arranged regularly and proteoglycan and others that exist to fill the gaps of the collagen fibers.
The collagen contains type I collagen as the major component, and also contain type IV collagen that maintains the equal interval arrangement of the collagen fibers. Such homogeneity of the collagen fibers ensures transparency of the cornea. In the case of a corneal edema or the like, opacity is caused by break of the homogeneity of the collagen fibers.
The keratocytes have long protrusions like extending tentacles to form a net-like structure in each of the layers. The keratocytes are larger in size and number and less dense at deeper positions from the corneal surface.
The Descemet's membrane is a basal membrane of the corneal endothelial cells and is a layer of about 30-40 $\mu$m.
The corneal endothelial cells are a single layer of cells located at the deepest part of the cornea. The corneal endothelial cells are uniformly arranged like cobblestones. The shape of the corneal endothelial cell is generally pentagonal, hexagonal or heptagonal, and mostly hexagonal. The corneal endothelial cell usually has a diameter of about 20 $\mu$m and has an area of about 300-350 $\mu$m$^2$.
The corneal endothelial cell of an adult hardly divides. When there is an abnormality of a cell, the surrounding cells expand, deform or move to repair the deficient. Such a structure makes the corneal endothelial cells prevent moisture from entering from the anterior chamber of the eye into the corneal stroma and function as a pump between the corneal stroma and the anterior chamber.

Next, a lesion of the cornea will be described. An endothelial dysfunction occurring after a cataract operation or the like decreases the pump function of the corneal endothelium and causes an edema in the corneal stroma. Due to the influence thereof, the morphology of the corneal stroma changes, and moreover, detachment of the Descemet's membrane occurs.

Further, in the ocular hypotension state, the water intake pressure increases due to the influence of the swelling pressure, and an edema is caused in the corneal stroma.

On the other hand, in the hypertension state, the swelling pressure decreases while the water intake pressure increases due to the ocular pressure. Consequently, moisture entering through the corneal endothelium passes through the corneal stroma but is blocked by the firmly joined superficial cells. As a result, an edema is caused in the corneal epithelial layer.

Further, under the condition of both endothelial dysfunction and hypertension, an edema of the corneal stroma, detachment of the Descemet's membrane, and an edema of the corneal epithelium may be caused simultaneously.

As described above, an edema is caused by accumulation of moisture that has entered the cornea. A mild edema is observed as a relatively small change in morphology, such as change of the cell size (density) and mild opacity due to disorder of uniformity. A severe edema is observed as a relatively large change in morphology, such as opacity and abrasion.

As described above, a lesion of the cornea is observed as a unique morphology to the cause of the lesion. The memory 247 previously stores association information 247b that associates identification information of a cell with the morphology of a cell as information for determining such a lesion.

The association information 247b includes an allowable range of the morphology of a cell of the cornea. This allowable range shows an allowable range of the morphology in a normal cell. The association information 247b associates identification information (name and so on) of a corneal cell with an allowable range of the morphology of the cell (normal morphology information). As described above, the morphology of a cell is, for example, the size of the cell (the thickness, width, cross-sectional area, volume, density, peripheral length, diameter, and so on), the shape of the cell (the ratio of the thickness and the width, a hexagonal shape, existence of a protrusion, and so on), and the junction morphology of the cell (tight junction, and so on).

It is sufficient that the association information 247b includes an allowable range of at least one of the various cells mentioned above, depending on the usage of the cornea observation device 100, and so on.

The association information 247b can be generated by, for example, clinically acquiring a number of morphology data on normal eyes and obtaining statistical values such as an average value, a median value and standard deviation. It is desirable to properly update the association information 247b when, for example, new clinical data is obtained.

(Cellular-Region Extracting Part)

A cellular-region extracting part 243 extracts an image region of a cell based on the pixel values of pixels composing an (horizontal) image of the cornea Ec. The cellular-region extracting part 243 is an example of the "extracting part" of the present invention.

In general, in a corneal image acquired by an OCT device, a boundary region of a cell has higher luminance and an internal region of the cell has lower luminance. This is because scattering at a boundary region of a cell is larger than scattering in an internal region.

Based on a threshold value previously set based on such a property, the cellular-region extracting part 243 can extract an image region of a cell by specifying an image region corresponding to a boundary region of a cell.

The process of extracting an image region of a cell is not limited to the above example, and it is possible to apply any publicly known technique for extracting a target region from an image. For example, it is possible to use a binarizing process, a filtering process, and so on.

The pixel values are luminance values in the case of a monochrome image and RGB values in the case of a color image. An image acquired by an OCT device is generally a monochrome image. A pseudo color image may be formed based on the distribution of luminance values.

(Cellular-Information Generator)

A cellular-information generator 244 generates cellular information based on an image region extracted by the cellular-region extracting part 243. The cellular-information generator 244 is an example of the "generator" of the present invention. The cellular information is information on a cell of the cornea Ec.

In this embodiment, morphology information of the cornea Ec is generated as the cellular information. The morphology information includes information such as the morphology of a cell of the cornea Ec, that is, the size, shape and so on of the cell.

To be specific, the cellular-information generator 244 generates the same kind of information as the morphology of the cell included in the relation information 247a. For example, in a case that the relation information 247a is information that relates the depth position and the area of the cell, the cellular-information generator 244 obtains, as the morphology information, the area of the cell based on the image region of the cell.

The size of a cell is calculated with reference to the magnification of an image. Information of the magnification is set at the time of acquisition of an image. When the magnification is already known, the scale of a distance (a unit distance) and the interval of pixels in an image can be acquired. The diameter and the periphery can be easily calculated based on the unit distance and the interval of pixels.

The area of an image region of a cell can be calculated by counting the number of pixels included in the unit area to acquire the unit area pixel number, counting the number of pixels within the image region of the cell, and dividing the number of pixels by the unit area pixel number. The area can also be obtained by executing an usual integration calculation.

On the other hand, in a case that the relation information 247a is information that relates the depth position to the shape of a cell, the cellular-information generator 244 obtains the shape of the cell based on the image region of the cell and sets it as the morphology information.

The shape of a cell (the horizontal cross-sectional shape or the like) can be obtained based on a wire model generated by thinning the image region of the boundary region of the cell described above.

Such a wire model generally includes boundary regions of a plurality of cells. A boundary region of a single cell can be specified by searching a looped image region that does not include part of the wire model.

Further, determination of the shape can be performed by calculating differential coefficient at each position on the looped image region, or can be performed by a pattern matching process or the like.

It is desirable to obtain the cellular information by executing a statistical process on image regions of a plurality of cells. Image regions of a number of cells exist in an image, and the image regions of the respective cells have various morphologies. Therefore, when morphology information is generated from the morphology of one of the cells, the morphology information is less reliable. Accordingly, it is desirable to obtain the morphologies of the respective image regions of the plurality of cells, execute a statistical process on the plurality of morphologies to obtain the average value, standard deviation (variance), the mode value, the median value or the like, and generate the cellular information based thereon.

In the case of considering the size of a cell, it is possible to obtain the size of each cell, calculate the average value or the like of the size, and adopt the average value or the like as the size of the cell for the image.

Further, in the case of considering the shape of the cell, it is possible to obtain the shape of each cell, obtain the most shape, and adopt the most shape as the shape of the cell for the image.

In a case that the morphologies of a plurality of cells are obtained, distribution information of the morphologies may be included in the morphology information. This distribution information may represent positional distribution of the cellular morphologies in the image, or may represent quantitative distribution such as a histogram in which the cellular morphologies are classified.

(Depth-Position Specifying Part)

A depth-position specifying part 245 specifies the depth position of an image in the cornea Ec based on the cellular information (morphology information) of the cornea Ec. To be specific, the depth-position specifying part 245 selects a depth position corresponding to the morphology of the cell of the cornea Ec represented by the morphology information from the relation information 247a, and sets it as the depth position of the image.

For example, since the corneal epithelial layer is composed of the basal cells, the wing cells and the superficial cells, which are different in size, it is possible to specify the depth position thereof by considering the size. Moreover, since most of the cornea endothelial cells are hexagonal, it is possible to specify the depth position by considering the shape.

(Abnormality Determining Part)

An abnormality determining part 246 determines whether there is an abnormality of a cell of the cornea Ec based on the cellular information of the cornea Ec. The abnormality determining part 246 is an example of the "determining part" of the present invention.

An example of a method for determining an abnormality of a cell will be described. As mentioned before, the memory 247 stores the association information 247b that associates identification information of a corneal cell with normal morphology information of the cell.

To the abnormality determining part 246, morphology information generated by the cellular-information generator 244 and a depth position specified by the depth-position determining part 245 are inputted. Here, the depth position is synonymous with cellular identification information. That is to say, one cellular identification information corresponds to one depth position, and one depth position corresponds to one cellular identification information.

The abnormality determining part 246 specifies normal morphology information corresponding to the specified cellular identification information. Next, the abnormality determining part 246 determines whether the morphology (size or shape) of cells of the cornea Ec represented in the generated morphology information is included in the normal morphology information (allowable range). If included, the abnormality determining part 246 determines the cell is normal. On the contrary, if not included, the abnormality determining part 246 determines there is an abnormality in the cell.

In a case that distribution information of morphologies of a plurality of cells is obtained as the morphology information, the abnormality determining part 246 can determine whether there is an abnormality based on the distribution information. For example, the abnormality determining part 246 can be configured to determine "abnormal" in a case that the number of cells having shapes other than a hexagonal shape exceeds a predetermined value in an image of the corneal epithelial layer. Moreover, the abnormality determining part 246 can be configured to, regarding the distribution of sizes of cells, determine "abnormal" in a case that the number of cells out of a normal size range exceeds a predetermined number.

[Operation Pattern]

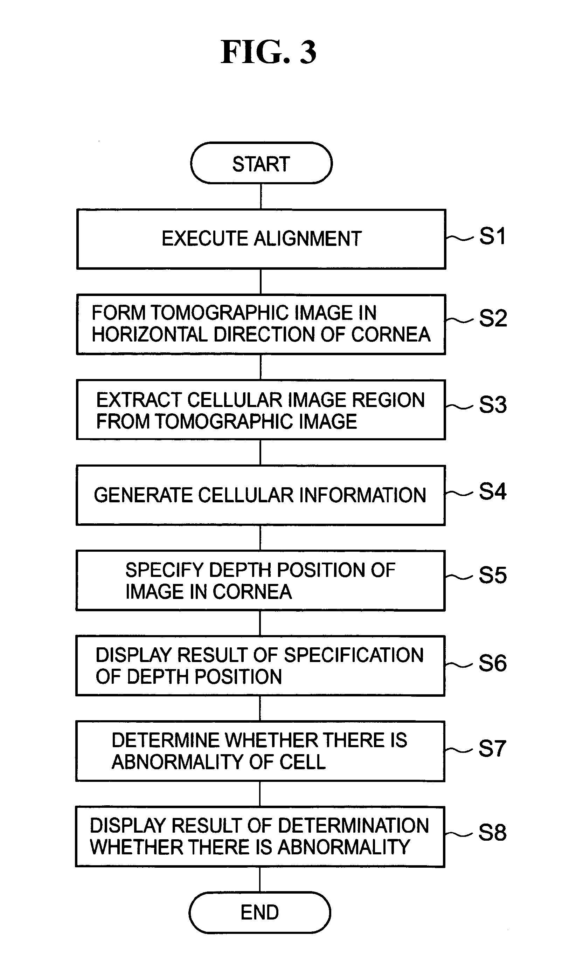

An operation pattern of the cornea observation device 100 will be described with reference to Fig. 3.

First, the eye E is placed at a predetermined measurement position, and alignment of a device optical system with respect to the eye E is executed (S1).

Next, a horizontal tomographic image of the cornea Ec is formed (S2). An operation of forming an image will be described below.

When the operator executes a predetermined manipulation for starting a measurement by the manipulation part 23, the controller 21 turns the halogen lamp 1 on. In this operation pattern, a continuous light of the broadband light M is emitted while the halogen lamp 1 is on.

Next, the controller 21 controls the reference-mirror moving mechanism 10 to set the optical path length of the reference light R to a first optical path length. The first optical path length corresponds to a first depth position (z coordinate value) of the cornea Ec. The controller 21 controls the exposure times of the CCDs 16 and 17. The CCDs 16 and 17 output the interference light detection signals $C_A$ and $C_B$, respectively.

Next, the controller 21 controls the reference-mirror moving mechanism 10 to switch the optical path length of the reference light R to a second optical path length. The second optical path length corresponds to a second depth position of the cornea Ec. The controller 21 controls the exposure times of the CCDs 16 and 17 to output new detection signals $C_A'$ and $C_B'$.

The first optical path length and the second optical path length are previously set so as to have a distance interval such that the detection signal $C_A$ and the detection signal $C_A'$ have a phase difference of 180° ($\pi$) and the detection signal $C_B$ and the detection signal $C_B'$ have a phase difference of 180° ($\pi$). Since the detection signals $C_A$ and $C_B$ have a phase difference of 90°, the four detection signals $C_A$, $C_B$, $C_A'$ and $C_B'$ for every phase difference of 90° are consequently obtained.

The image forming part 241 adds the detection signals $C_A$ and $C_A'$ (a phase difference of 180°) and dividing the sum by 2, thereby calculating a background light component $I_s(x,y)+I_r(x,y)$. This calculation process may be executed by using the detection signals $C_B$ and $C_B'$ (a phase difference of 180°).

Furthermore, the image forming part 241 subtracts the background light component $I_s(x,y)+I_r(x,y)$ from the respective detection signals $C_A$ and $C_B$, thereby obtaining interference components (cos component, sin component). Then, the image forming part 241 calculates the sum of squares of the interference components of the respective detection signals $C_A$ and $C_B$, thereby forming an image in a cross section of the xy-direction (horizontal direction). This process may be executed with the detection signals $C_A'$ and $C_B'$ (having a phase difference of 180°).

The controller 21 repeats the above process while changing the optical path length of the reference light R, thereby sequentially forming images in the xy-cross-section at various depth positions of the cornea Ec. Consequently, it is possible to obtain images at various depth positions such as the superficial cell, the wing cell, the basal cell, the Bowman's layer, and the corneal stroma.

In this process, the controller 21 controls the CCDs 16 and 17 to output detection signals at a predetermined frame rate and at the same timings, and also causes this frame rate, the exposure timings of the CCDs 16 and 17, the movement timing of the reference mirror 9 and the change timing of the optical path length of the reference light R to synchronize.

In this case, the exposure times of the CCDs 16 and 17 are set shorter than the frame rate. For example, it is possible to set the frame rate of the CCDs 16 and 17 to 30 f/s and set the exposure times to approximately 30~50 $\mu$s.

Further, it is possible to acquire an image having resolution of about several $\mu$m, by using the broadband light M whose central wavelength is about 760 nm and the wavelength width is about 100 nm. For example, assuming the wavelength of the broadband light M is Gaussian, a theoretical value of the resolution when the refractive index of the eye E is n=1.33 is about 1.8 $\mu$m.

An image of the cornea Ec thus acquired is stored into the memory 247, for example.

Further, the controller 21 controls the display 22 to display a horizontal tomographic image of the cornea Ec in response to manipulation through the manipulation part 23, for example.

When a tomographic image of the cornea Ec is formed, the cellular-region extracting part 243 extracts an image region of a cell based on the pixel values of the pixels composing this tomographic image (S3).

Next, the cellular-information generator 244 generates the cellular information (morphology information) based on the extracted image region (S4).

Subsequently, the depth-position specifying part 245 specifies the depth position of the image in the cornea Ec based on the cellular information (morphology information) of the cornea Ec and the relation information 247a (S5).

The controller 21 controls the display 22 to display the result of the specification of the depth position (S6). This specification result is a string representing the name of a layer of the cornea, for example. Further, it is also possible to display a model image of a cross section in the depth direction of the cornea (an image that depicts the various layers) and explicitly display a layer of the specification result. Thus, the operator can grasp the depth position of the image (the tomographic image along the horizontal direction.

Next, the abnormality determining part 246 determines whether there is an abnormality of a cell of the cornea Ec based on the depth position and the cellular information of the cornea Ec (S7).

The controller 21 controls the display 22 to display the result of the determination whether there is an abnormality (S8). This determination result is, for example, a string or image that represents the determination result. In particular, when the determination result is "abnormal," audio information such as an alarm sound may be outputted. This enables the operator to grasp whether there is an abnormality of the cell at the depth position. This is the end of the description of the operation pattern of the cornea observation device 100.

[ACTION AND EFFECT]

The action and effect of the cornea observation device 100 will be described.

The cornea observation device 100 forms an image of a cell of the cornea Ec and analyzes the morphology of the cell shown in this image, thereby being capable of specifying the depth position of the image in the cornea Ec. Therefore, according to the cornea observation device 100, it is possible to grasp the depth position of an image in the cornea Ec.

To be specific, as the depth position in the cornea, it is possible to specify the kind of a cell shown in an image, that is, the layer of the cell. Such a specification is realized by utilizing a fact that the cornea has the aforementioned multilayer structure and the kind of a cell differs depending on the depth of a layer. By thus specifying the layer of a cell, it is possible to grasp what layer or membrane of the cornea has been imaged.

Further, the cornea observation device 100 is configured to generate the cellular information (morphology information) based on the image region of a cell in an image and specify the depth position of the image based on this cellular information. Therefore, it is possible to specify the depth position of an image with high accuracy by utilizing a characteristic morphology of a cell.

Further, according to the cornea observation device 100, since it is possible to generate the cellular information by executing a statistical process on image regions of a plurality of cells, it is possible to generate highly accurate cellular information and specify the depth position of an image with high accuracy.

Further, according to the cornea observation device 100, it is possible to determine whether there is an abnormality of a cell of the cornea Ec based on the cellular information. Consequently, it is possible to support a medical examination of the cornea.

Further, since an image obtained by the cornea observation device 100 depicts a cell-level minute structure, it is possible to expect early detection of a corneal disease by enabling detection of an abnormality at the cell level.

The result of determination of an abnormality by the cornea observation device 100 is not a conclusive diagnosis result, and merely indicates the possibility of existence of an abnormality after all. A final diagnosis result is determined by a doctor based on images and results of other examinations.

In order to detect a cell-level abnormality, the cornea observation device 100 acts to determine whether there is an abnormality of a cell by previously storing an allowable range of the morphology of a cell of the cornea (the association information 247b) and determining whether the morphology of cells of the cornea Ec obtained by an examination is included in the allowable range.

Further, the cornea observation device 100 is equipped with an OCT device using an interferometer and is therefore capable of a measurement with high resolution and high sensitivity. Moreover, the cornea observation device 100 uses a weak broadband light as an illumination light and is therefore highly safe for the eye E.

Furthermore, the cornea observation device 100 is equipped with a full-filed OCT device and is therefore capable of acquiring an image of higher resolution than an image acquired by another device. Thus, the cornea observation device 100 has an advantage in that it is possible to quite closely observe the minute structure of the cornea Ec.

Further, according to the cornea observation device 100, since it is possible to simultaneously acquire the two polarization components L1 and L2 of the interference light L, it is possible to shorten a measurement time. To be specific, since the cornea observation device 100 is configured to form an image by acquiring the four detection signals $C_A$, $C_B$, $C_A'$ and $C_B'$ having different phases in two measurements, it is possible to shorten a measurement time.

Further, since it is possible to easily and rapidly switch acquisition of the detection signals $C_A$ and $C_B$ and acquisition of the detection signals $C_A'$ and $C_B'$ only by switching the optical path length of the reference light R, it is possible to shorten a measurement time.

As mentioned before, the state of reflection and scattering of the signal light S by the cornea Ec is reflected on the level of a luminance value. Therefore, it is also possible to apply a configuration provided with only one CCD, instead of disposing two CCDs as in this embodiment. In this case, there is no need to convert the polarization property or divide polarization components (in other words, a configuration therefor is unnecessary). The configuration with two CCDs is favorable for an examination of a living eye because it has such a merit that a measurement time can be shortened as mentioned later. On the other hand, the configuration with one CCD has such a merit that the configuration of the device can be simplified.

Further, according to the cornea observation device 100, by changing a difference in optical path length between the reference light R and the signal light S, it is possible to easily acquire images at various depth positions of the cornea Ec. In particular, by executing a measurement as in the operation pattern described above, it is possible to rapidly acquire images at various depth positions.

According to the cornea observation device 100, in the case of acquiring tomographic images at a plurality of depth positions, it is possible to specify the depth positions of the respective tomographic images. For this, it is possible to execute the aforementioned process on each of the tomographic images to specify the depth position. Moreover, in a case that an interval between adjacent tomographic images is already known, it is possible to specify the depth position of one of the tomographic images by the aforementioned process and specify the depth position of the other tomographic image based on the specified depth position and the interval between the images.

Further, according to the cornea observation device 100, it is possible to simultaneously detect the two polarization components L1 and L2 of the interference light L and there is no difference in detection time of the two polarization components L1 and L2, so that it is possible to form a highly accurate image without an influence by movement of the eye E.

Further, there is such a merit that use of the non-polarized broadband light M facilitates configuration of an optical system. That is to say, it is possible to facilitate configuration of an optical system by using the non-polarized broadband light M though, in the case of using a polarized broadband light such as a linearly-polarized light, there is a problem that the polarization state of the broadband light is affected when the light is propagated through a beam splitter or a lens and therefore configuration of an optical system for maintaining a polarization state is difficult.

Further, by using a thermal light source as a light emitting part and using an optical fiber bundle, it is possible to easily obtain a non-polarized broadband light. In the case of using a laser light source that emits a randomly-polarized broadband light, it is possible to easily obtain a randomly-polarized broadband light.

Further, by disposing the glass plate 7 as a dispersion correction optical element that minimizes the influence of dispersion occurring on the optical paths of the signal light S and the reference light R (both the arms of the interferometer), it is possible to resolve a difference in dispersion between the signal light S and the reference light R, and it is possible to efficiently acquire the interference light L on which information included in the signal light S is favorably reflected.

Further, by executing a measurement while setting the exposure times of the CCDs 16 and 17 short, it is possible, even when the eye E moves during the measurement, to form a highly accurate image without being influenced by the movement.

[MODIFICATION]

The embodiment described above is merely a specific example for implementing the cornea observation device according to the present invention. Therefore, it is possible to properly apply any modification within the scope of the present invention. In the following description, components similar to those of the aforementioned cornea observation device 100 will be denoted by the same reference numerals.

[First Modification]

Although the morphology information that represents the morphology of a corneal cell is used as the cellular information in the above embodiment, the cellular information is not limited thereto. In this modification, an example using other cellular information will be described.

The cellular-region extracting part 243 extracts an image region of a cell from an image of the cornea Ec as in the above embodiment. Based on this image region, the cellular-information generator 244 generates density information that represents the density of cells of the cornea Ec and sets it as the cellular information.

As mentioned before, the various cells of the cornea have characteristic sizes (cross-sectional areas in the horizontal direction), respectively. The density information is acquired by obtaining the number of cells within a unit area in an image. This process is realized by properly setting a region of a unit area in an image and counting the number of cells within this region. Moreover, it is also possible to realize by obtaining the area of any region (for example, the entire image) in an image, counting the number of cells within the region, and dividing the counted value by the area.

Also in the case of generating the density information, it is desirable to obtain the cell density in various regions in an image and statistically process.

Further, the relation information 247a in this modification is information that relates the depth position in the cornea to the density of cells. This relation information 247a is also previously generated and stored into the memory 247.

The depth-position specifying part 245 selects a depth position corresponding to the density represented by the density information of the cornea Ec from the relation information 247a, and setting this selected depth position as the depth position of the image.

This configuration makes it possible to grasp the depth position of an image in the cornea Ec. In particular, by using a characteristic cell density, it is possible to specify the depth position of an image with high accuracy.

[Second Modification]

The cornea observation device 100 according to the above embodiment is configured to, based on a tomographic image in a cross-section orthogonal to the depth direction of the cornea (a horizontal tomographic image), specify the depth position of the cornea shown in this tomographic image and determine whether there is a cell-level abnormality.

In this modification, a cornea observation device that, based on a tomographic image in a cross-section along the depth direction of the cornea (a depthwise tomographic image), specifies the depth position and determines whether there is an abnormality will be described. The depthwise tomographic image is an image in a cross-section orthogonal to the horizontal tomographic image.

The cornea observation device of this modification is configured similarly to that of the above embodiment. That is to say, this cornea observation device is capable of acquiring horizontal tomographic images at various depth positions of the cornea, by moving the reference mirror 9.

In a case that horizontal tomographic images at a plurality of depth positions are acquired, the image forming part 241 forms a depthwise tomographic image based on these tomographic images.

An example of this process will be described. Firstly, the image forming part 241 interpolates a pixel between adjacent horizontal tomographic images as required. Next, the image forming part 241 generates volume data based on the plurality of horizontal tomographic images. The volume data is image data defined by voxels, which are three-dimensional pixels. Subsequently, the image forming part 241 selects voxels located in a cross-section along the depth direction from the volume data, and forms a targeted tomographic image based on these voxels. Instead of generating the volume data, it is also possible to form a depthwise tomographic image based on image data in which a plurality of horizontal tomographic images are arranged in a three-dimensional coordinate system (called stack data or the like). The process described here is publicly known.

The cross-sectional position in the depth direction may be designated by the image forming part 241, or may be manually designated by the operator. In the former case, it is possible to automatically set a cross-sectional position designated in the past as in, for example, a third modification described later. Moreover, it is also possible to automatically set a predetermined cross-sectional position (for example, a cross-section passing through the corneal apex). On the other hand, in the latter case, it is possible to configure to make the display 22 display a pseudo three-dimensional image obtained by rendering volume data and to set a cross-sectional position by using the manipulation part 23 on this three-dimensional image.

The memory 247 previously stores the relation information 247a that relates the morphology of a depthwise cross-section of the cornea to the depth position of the cornea. This relation information 247a relates, for example, the thickness and arrangement pattern of cellular layers in the cross-section (the morphology of arrangement of layers) to the depth position of the cornea. Moreover, it is also possible to use the relation information 247a that relates a distance (depth) from a characteristic depth position of the cornea to the identification information of a cellular layer. Moreover, the relation information 247a may be one that relates the morphology of a cell in a depthwise cross-section (the size and shape of a cross-section) to the depth position of the cornea. As the characteristic depth position of the cornea, it is possible to adopt the corneal surface, the corneal endothelial layer, or the like.

Further, in the memory 247, the association information 247b is also previously stored. The association information 247b includes, for example, an allowable range of the morphology of a cell in a depthwise cross-section (the size and shape of the cross-section). Moreover, the association information 247b may include an allowable range of the morphology of a cellular layer in a depthwise cross-section (the thickness and arrangement pattern of the layer). As the allowable range of the arrangement pattern, for example, the number of layers is used.

The cellular-information generator 244 generates cellular information that includes morphology information of the cross-section of a cell shown in a depthwise tomographic image (the size, shape and so on) and morphology information of a cellular layer shown in this tomographic image (the thickness, arrangement pattern and so on of the layer). The generation method is similar to that of the above embodiment.

The depth-position specifying part 245, based on the morphology information included in the cellular information and the relation information 247a, specifies the depth position of a cellular layer (at least one) depicted in the tomographic image. This process can be executed in a similar manner to that in the above embodiment.

Instead of specifying the depth position based on a depthwise tomographic image, it is also possible to specify the depth position based on the measurement position (the position of the reference mirror 9) of the original horizontal tomographic image. This process can be executed based on association of a two-dimensional coordinate system in which the horizontal tomographic image is defined and a three-dimensional coordinate system in which stack data and volume data are defined (embedding of the former into the latter).

The abnormality determining part 246 firstly selects an allowable range corresponding to the specified depth position from the association information 247b. Next, the abnormality determining part 246 determines whether information on the morphology of a cross section of a cell of a cellular layer and the morphology of the cellular layer included in the cellular information are included in the allowable range. Consequently, it is determined whether there is an abnormality in the cellular layer.

According to this modification, it is possible to specify the depth position of a cellular layer in a depthwise tomographic image, based on the morphology of a cell in a depthwise cross-section.

Further, according to this modification, it is possible to determine whether there is an abnormality of the cornea, based on the morphology of a cell in a depthwise cross-section.

By executing the process according to this modification together with the process of the above embodiment, it is possible to specify the depth position from the morphology of a cell in both the horizontal cross-section and the depthwise cross-section, and therefore, it is possible to increase the accuracy of the process for specifying the depth position. Similarly, it is possible to increase the accuracy of determination whether there is an abnormality of the cornea.

Although the modifications using a depthwise tomographic image have been described above, it is also possible to execute a similar process by using a tomographic image in any cross-sectional direction. Designation of the cross-section can be executed by automatically or manually as in the case of using a depthwise tomographic image.

Further, it is also possible to previously store the relation information 247a and the association information 247b regarding the cross-sectional morphology of a cell and the morphology of a cellular layer in a plurality of cross-sectional directions. In this case, by selecting information corresponding to the cross-sectional direction and executing a similar process to the process described above by using the information, specification of the depth position and determination whether there is an abnormality are executed.

[Third Modification]

A cornea observation device according to this modification determines whether there is an abnormality of the cornea by comparing cellular information acquired at different times and dates (at least at different times). This modification is effective in the case of executing examinations plural times on the same eye such as an observation of the clinical course.

The cornea observation device according to this modification is provided with a storing part that stores the cellular information. As the storing part, for example, the memory 247 is used. Moreover, it is possible to use a memory device connected to the cornea observation device via a network, as the storing part. Moreover, it is possible to use a media (and a driver) as the storing part. As the media, it is possible to use any storage medium such as an optical disk, a magnetic disk and a semiconductor memory. The cornea observation device is equipped with a driver compliant with the used media.

The cellular information is stored into the memory 247 together with supplementary information such as a patient ID, an examination time and date, the result of specification of the depth position and the result of determination of an abnormality. The information including the supplementary information will be referred to as "cellular information."

When the cellular-information generator 244 generates new cellular information, the depth-position specifying part 245 specifies the depth position of the cornea represented in the image based on the new cellular information.

Next, the abnormality determining part 246 searches, for example, cellular information supplemented with the same patient ID as the patient ID (already inputted) relating to the new cellular information, from the memory 247. The cellular information to be searched is cellular information of the same depth position as that of the new cellular information.

In a case that a plurality of cellular information are searched, the controller 21 controls the display 22 to display a list of the search result, for example. In this case, the controller 21 controls to display the search result in order of time and date (ascending order/descending order) with reference to the examination time and date included in each supplementary information. It is sufficient that information to be displayed includes the examination time and date. The operator manipulates the manipulation part 23 to designate cellular information to be compared with the new examination result.

Instead of thus manually designating the comparison object, it is possible to configure to automatically select cellular information acquired in the last examination (the examination in which an image at the same depth position as the new cellular information has been acquired). This is because the last examination result is often compared with the present examination result in a follow-up or the like.

The abnormality determining part 246 acquires previous cellular information to be a comparison object from the memory 247. Then, the abnormality determining part 246 compares this previous cellular information with new cellular information and determines a change in morphology of the cornea.

In this process, for example, it is determined whether there is an abnormality based on the respective cellular information, and a change of a cell at the depth position is determined depending on whether the determination results of the both are the same or different. In a case that the previous determination result is "normal" and the new determination result is "abnormal," it is possible to determine that the state of the cell has worsened. In the contrary case, it is possible to determine that the state of the cell has improved. In other cases, it is possible to determine that the state of the cell has not changed. In a case that the supplementary information of the previous cellular information includes the determination result whether there is an abnormality, there is no need to redetermine this time.

In another determination process, it is possible to determine a change of the level of an abnormality of a cell as a change of the cell at the depth position. To be specific, firstly, based on the respective cellular information and the association information 247b, the level of an abnormality of the morphology of a cell is obtained. In this process, for example, a displacement from an allowable range of the morphology (size, shape) of a cell shown in cellular information is obtained. Next, by comparing the past displacement and the present displacement, a change of the level of the abnormality of the cell at the depth position is determined. In this process, for example, by comparing the past displacement and the present displacement, a temporal change of the cell is determined.

According to this modification, it is possible to easily determine a temporal change of the morphology of a cell. Consequently, it is possible to determine a change of the level of an abnormality of a cell, for example, the level of advance of the clinical condition and the level of a treatment effect.

[Fourth Modification]

A cornea observation device according to this modification is configured to specify the depth position of an image in the cornea based on the pixel values of pixels composing the image.

In an image acquired by the cornea observation device, an abnormal site and a normal site in the cornea are depicted in different patterns. For example, a site where an edema is caused is depicted lighter (namely, with a higher luminance value) than a normal site. This modification describes detection of an abnormality of the cornea by focusing on such a difference in pixel value.

For this purpose, the relation information 247a that relates the depth position in the cornea to the pixel values of an image is previously stored in the memory 247. The relation information 247a is generated, for example, based on clinically acquired data. The relation information 247a is defined as, for example, a threshold value (an allowable range or the like) of the pixel value.

Further, the association information 247b that associates the depth position in the cornea with the allowable range of the pixel value is previously stored in the memory 247. The association information 247b is generated, for example, based on clinically acquired data. For example, the association information 247b can be defined as a threshold value (an allowable range or the like) of the pixel value, or can be defined as a comparison value (a ratio or the like) between the pixel value of a normal site and the pixel value of an abnormal site.

The analyzer 242 analyzes a horizontal tomographic image of the cornea Ec, selects a depth position corresponding to the pixel values of pixels composing this tomographic image from the relation information 247a, and sets it as the depth position of the tomographic image in the cornea Ec.

In this process, for example, like the aforementioned cellular-region extracting part 243, it is possible to extract an image region of a cell from a tomographic image and obtain a target pixel value by using the pixel value of a pixel within this image region (a boundary region or internal region of a cell). In this case, the relation information 247a is previously formed so as to associate the pixel value of an image region to be extracted with the depth position of the cornea.

Further, it is also possible to execute a statistical process on the pixel values of pixels composing an image and specify the depth position of the image based on the result.

Further, the analyzer 242 (the abnormality determining part 246) can also determine whether there is an abnormality of a cell of the cornea Ec based on the pixel values of pixels composing an image. This determination process can be implemented by acquiring an allowable range or the like corresponding to a specified depth position and determining whether the pixel values of the image (or a partial region thereof) are included in the allowable range or the like.

According to this modification, instead of a characteristic morphology of a cell as in the above embodiment, it is possible to specify the depth position by considering the pixel values of pixels. Moreover, according to this modification, it is possible to detect an abnormality, such as an edema, of the cornea reflected on the pixel values.

[Anther Modification]

Although a configuration to determine whether there is an abnormality of the cornea has been chiefly described above, it is also possible to determine the level of an abnormality. In this modification, the abnormality determining part 246 is configured to obtain a displacement from an allowable range of the morphology (size, shape) of a cell and determine the level of the abnormality based on the level of this displacement.

In this process, it is possible to regard the level of the displacement as the level of the abnormality, or it is possible to previously associate the level of a displacement with the level of an abnormality (the association information 247b) and obtain the level of the abnormality based on the association.

It is possible to determine in consideration of not only the level of a displacement but also the direction of the displacement (larger/smaller than an allowable range).

A cornea observation device according to the present invention may be provided with a measuring part that measures the size of a cell determined to have an abnormality. The measuring part is configured to include the analyzer 242, for example. In this measuring process, for example, a unit distance (for example, an interval between pixels) in an image based on the magnification or the like of the image is obtained and the size of a cell is obtained based on this unit distance. In this process, the size of one cell may be obtained, or the size of the entire region of cells determined to have an abnormality.

The cornea observation device according to the present invention may be provided with an output part that outputs the depth position specified by the depth-position specifying part 245 and the result of determination by the abnormality determining part 246. The output part is, for example, the display 22 that displays the depth position and the determination result. Moreover, the controller 21 may output the information to another device via a network. Moreover, it is possible to output and record the information into a media such as a CD-R (a driver is installed). Moreover, it is possible to print out the information by a printer.

As described in the above embodiment and modifications, according to the present invention, it is possible to analyze the morphologies of various kinds of tissues forming the cornea. By relating the analysis results of the morphologies with diagnosis results and accumulating them, it is possible to grasp a standard change in morphology of the corneal tissues in each of the various diseases. In this case, it is desirable to analyze a number of cases and execute a statistical process to increase the reliability of information. Furthermore, clinical conditions (seriousness of a disease, complication, and so on) in the respective cases may be considered. Moreover, by following a change with time in each of the cases, information of a standard change with time of the morphologies of the corneal tissues may be acquired.

A specific example of such a modification will be described. As a disease that the nerve fiver plexus of the Bowman's layer become short, HIV (human immunodeficiency virus), diabetes and so on are known. Besides, it is known that the number of branches of the nerve fiber plexus decreases in the case of diabetes.

Therefore, a change in length of the nerve fiber plexus (the actual length, the shortening rate or the like) and the result of diagnosis (the name of the disease, such as HIV infection and diabetes) are related to each other and stored. Consequently, information on a standard change in length of the nerve fiber plexus is obtained for each of various kinds of diseases such as HIV infection and diabetes.

Similarly, by obtaining the number of the branches of the nerve fiber plexus and relating a change in number of the branches to the diagnosis result to store, information on a standard change in number of the branches of the nerve fiber plexus in each of various kinds of diseases can be obtained.

For a disease like diabetes in which plural kinds of morphological changes in the morphology of the corneal tissues are caused, it is possible to obtain more comprehensive information of morphological changes by relating the plural kinds of morphological changes to diagnosis results and storing them.

If information of the morphological change of the corneal tissues is thus generated and stored for each of various diseases, it is possible to extract potential diseases that may be affecting the patient, by comparing the result of analysis of an image of a cell of the cornea newly obtained with the information. The corneal observation device outputs the extracted potential diseases. The outputted potential diseases can be utilized for diagnosis support.

When analyzing the morphology of tissues forming the cornea, it is possible to use image processing such as pattern matching. For example, the corneal keratocytes have a net-like structure as mentioned before and, as the depth from the corneal surface increases, the size increases, the number increases and the density gets rougher. Therefore, in the case of the cornea that has an abnormality in thickness of a layer, it may be impossible to, by merely analyzing a magnified image of the keratocytes, accurately grasp a depth position shown by the image. For example, in a case that the layer has become thick due to an edema, there is a case that the keratocytes of the expected size do not exist at the normal depth position.

In order to deal with such a case, a standard arrangement pattern of the keratocytes in a horizontal cross-section or depthwise cross-section is previously stored. Then, the arrangement pattern of the keratocytes depicted in an image of the cornea and the standard arrangement pattern are compared by pattern patching. Alternatively, the arrangement pattern of the keratocytes depicted in a horizontal tomographic image acquired in the past and the arrangement pattern of the keratocytes depicted in a horizontal tomographic image newly acquired are compared by pattern matching.

By executing such a process, it is possible to determine whether the reason for change of the size or density of the keratocytes is simple difference of a measurement depth or partial change of the thickness of a layer due to an edema or the like. In particular, in the latter case, it is possible to present an image expressing change of the thickness of a layer. This image is, for example, an image that depicts change of the thickness of a layer by color-coding, and an image that depicts by gradation. Further, it is also possible to present an image that explicitly shows a site where the thickness of a layer has changed, or a site where the change is much.

The cornea observation device according to the present invention may be provided with an image forming part and an analyzer. The image forming part radiates a light to an eye, detects the light propagated though the cornea, and forms an image of a corneal cell based on the result of this detection.

The image forming part is configured, for example, similarly to the image forming part 241 of the above embodiment. The analyzer analyzes the morphology of the cell shown in the image formed by the image forming part, and determines whether there is an abnormality of the corneal cell.

The analyzer is configured by, for example, the analyzer 242 of the above embodiment. However, in this configuration, the analyzer does not need to be provided with the depth-position specifying part 245.

In the case of use of this cornea observation device, an image at a certain depth position of the cornea is acquired. For this purpose, for example, the operator adjusts the position of the reference mirror 9 so that an image at a certain depth position is acquired.

By executing such an adjustment and so on, it is possible to determine whether there is an abnormality of the cell at the depth position without specifying the depth position of the cornea shown in the image.

The aforementioned cornea observation device 100 is configured to convert the polarization property of the reference light R, but may convert the polarization property of the signal light S. In this case, a waveplate (a $\lambda/4$ plate), a polarization plate and a glass plate are disposed on the optical path of the signal light S.

The aforementioned cornea observation device 100 converts the polarization property by using the waveplate 4 and the polarization plate 5, but may use any type of converter capable of converting the polarization property. Further, although the reference light R is converted into a circularly-polarized light in the aforementioned configuration, it is also possible to configure to convert the reference light R or the signal light S so as to have any polarization property (linear polarization, elliptical polarization), depending on the configuration of an optical image forming device.

Although dispersion caused on both the arms of the interferometer is corrected by the glass plate 7 in the aforementioned cornea observation device 100, it is also possible to apply a dispersion correction optical element like an optical element of any type capable of correcting the dispersion.

Although the CCDs 16 and 17 are used as the detector in the aforementioned cornea observation device 100, it is possible to apply any two-dimensional light sensor array such as CMOS as the detector.

The aforementioned cornea observation device 100 responds to the movement of the eye E, and so on, by using a continuous light of a broadband light and making the exposure times of the CCDs 16 and 17 short, but the configuration thereof is not limited to this configuration.

For example, it is possible to dispose a light chopper on the optical path of the broadband light (continuous light), periodically interrupt the broadband light by this light chopper to generate pulse-like broadband lights, and detect the respective pulses by the CCDs 16 and 17.

The interrupting period of the broadband light by the light chopper is about 1 ms, which is longer than the exposure time (about 30-50 $\mu$s). Therefore, in a case that the movement of the eye E is rapid, it is desirable to control the exposure time.

Further, it is possible to configure to output a broadband light composed of flush lights by using a light source such as a xenon lamp and detect the respective flush lights by the CCDs 16 and 17.

Further, the aforementioned cornea observation device 100 is configured to acquire the two detection signals $C_A$ and $C_B$ ($C_A$' and $C_B$') having a phase difference of 90° in one measurement, but may be configured to acquire two detection signals having a phase difference of 180° by using a $\lambda/2$ plate as the waveplate 4, for example. In this case, a first optical path length and second optical path length of the reference light R are previously set so as to be such a distance interval that a detection signal obtained in a first detection process and a detection signal obtained in a second detection process have a phase difference of 90°. Consequently, it is possible to acquire four detection signals at every phase difference of 90°.

Although the optical image measurement device provided with a Michelson interferometer has been described in the above embodiment and so on, it is needless to say that another interferometer such as a Mach-Zehnder interferometer can be employed.

Further, by disposing an optical fiber (bundle) to part of the interferometer and using as a light-guiding member, it is possible to increase the degree of freedom of the device design, reduce the size of the device, or increase the degree of freedom of placement of a measurement object.

The cornea observation device according to the present invention may be any combination of the configurations of the embodiment and modifications described above. With combination of the configurations, a cornea observation device that has at least actions and effects of combination of actions and effects unique to the respective configurations is formed.

**Claims**

1. A cornea observation device, comprising:

   an image forming part configured to radiate a light to an eye, detect the light propagated though a cornea, and form an image of a cell of the cornea based on a result of the detection; and
   a specifying part configured to analyze a morphology of the cell shown in the image and specify a depth position of the cornea shown in the image.

2. The cornea observation device according to Claim 1, wherein the specifying part is configured to specify a layer of the cornea shown in the image as the depth position.

3. The cornea observation device according to Claim 1, wherein the specifying part includes an extracting part configured to extract an image region of at least one cell in the image and a generator configured to generate cellular information on the morphology of the cell of the cornea based on the image region, and is configured to specify the depth position of the image based on the cellular information.

4. The cornea observation device according to Claim 3, wherein the specifying part includes a determining part configured to determine whether there is an abnormality of the cell of the cornea based on the cellular information.

5. The cornea observation device according to Claim 4, wherein:

   the image forming part is configured to form a two-dimensional image of the cornea in a cross-section substantially orthogonal to a depth direction of the cornea;
   the generator is configured to generate the cellular information including morphology information of a cross-section of a cell shown in the two-dimensional image; and
   the determining part is configured to determine whether there is an abnormality of the cell in the cross-section based on the morphology information.

6. The cornea observation device according to Claim 4, wherein:

   the image forming part is configured to generate two-dimensional images of the cornea in a plurality of cross-sections substantially orthogonal to a depth direction of the cornea, respectively, and form a tomographic image in a cross-section along the depth direction based on the plurality of two-dimensional images;
   the generator is configured to generate the cellular information including morphology information of a cross-section of a cell shown in the tomographic image and/or morphology information of a cell layer shown in the tomographic image; and
   the determining part is configured to determine whether there is an abnormality of a cell in the cross-section along the depth direction based on the morphology information.

7. The cornea observation device according to Claim 4, wherein the determining part is configured to previously store an allowable range of a morphology of a cell of a cornea, determine whether the morphology represented in the cellular information is included in the allowable range, and determine the cell of the cornea is normal when determining the morphology is included, whereas determine the cell of the cornea is abnormal when determining the morphology is not included.

8. The cornea observation device according to Claim 7, wherein the determining part is configured to previously store the allowable range at each of a plurality of depth positions of a cornea, and select the allowable range corresponding to the depth position specified by the specifying part to execute the determination.

9. The cornea observation device according to Claim 4, further comprising a storing part configured to store the cellular information, wherein the determining part is configured to, when new cellular information is generated by the generator, compare the cellular information previously stored in the storing part with the new cellular information and determine a change of the morphology of the cell of the cornea.

10. The cornea observation device according to Claim 3, wherein the specifying part includes a memory part configured to previously store relation information that relates a depth position in a cornea to a morphology of a cell, and is configured to select a depth position corresponding to the morphology represented in the cellular information from the relation information and set the selected depth position as the depth position of the image.

11. The cornea observation device according to Claim 3, wherein the morphology of the cell includes a size and/or shape of a cell of a cornea.

12. The cornea observation device according to Claim 3, wherein:

the generator is configured to generate density information that represents a density of the cell of the cornea based on the image region, as the cellular information; and
the specifying part is configured to specify the depth position of the image based on the density information.

13. The cornea observation device according to Claim 12, wherein the specifying part includes a memory part configured to previously store relation information that relates a depth position in a cornea to a density of a cell, and is configured to select a depth position corresponding to the density represented in the density information from the relation information and set the selected depth position as the depth position of the image.

14. The cornea observation device according to Claim 3, wherein:

the extracting part is configured to extract image regions of a plurality of cells; and
the generator is configured to execute a statistical process on the image regions of the plurality of cells and generate the cellular information.

15. The cornea observation device according to Claim 1, wherein the specifying part is configured to analyze the morphology of the cell based on pixel values of pixels composing the image and to specify the depth position of the image.

16. The cornea observation device according to Claim 15, wherein the specifying part includes a memory part configured to previously store relation information that relates a depth position in a cornea to pixel values of pixels composing an image of the cornea, and is configured to select a depth position corresponding to pixel values of pixels composing the image formed by the image forming part from the relation information and to set the selected depth position as the depth position of the image.

17. The cornea observation device according to Claim 15, wherein the specifying part is configured to execute a statistical process on pixels values of pixels composing the image and specify the depth position of the image based on a result of the statistical process.

18. The cornea observation device according to Claim 15, wherein the specifying part includes a determining part configured to determine whether there is an abnormality of the cell of the cornea based on pixel values of pixels composing the image.

19. The cornea observation device according to Claim 4, provided with an output part configured to output a result of determination by the determining part and the depth position of the image specified by the specifying part.

20. The cornea observation device according to Claim 18, provided with an output part configured to output a result of determination by the determining part and the depth position of the image specified by the specifying part.

21. The cornea observation device according to Claim 4, wherein the specifying part includes a measuring part configured to measure a size of a cell determined to have an abnormality by the determining part.

22. The cornea observation device according to Claim 18, wherein the specifying part includes a measuring part configured to measure a size of a cell determined to have an abnormality by the determining part.

23. A cornea observation device, comprising:

an image forming part configured to radiate a light to an eye, detect the light propagated though a cornea, and form an image of a cell of the cornea based on a result of the detection; and
an analyzer configured to analyze the morphology of the cell shown in the image and to determine whether there is an abnormality of the cell of the cornea.

24. The cornea observation device according to Claim 1, wherein the image forming part is an OCT device including an interference-light generator configured to split a broadband light into a signal light and a reference light and superimpose the signal light propagated through a cornea and the reference light propagated through a reference object to generate an interference light, a detector configured to detect the interference light, and a forming part configured to form an image of a cell of the cornea based on a detection result of the interference light.

25. The cornea observation device according to Claim 23, wherein the image forming part is an OCT device including an interference-light generator configured to split a broadband light into a signal light and a reference light and superimpose the signal light propagated through a cornea and the reference light propagated through a reference object to generate an interference light, a detector configured to detect the interference light, and a forming part configured to form an image of a cell of the cornea based on a detection result of the interference light.

26. The cornea observation device according to Claim 24, wherein:

the interference-light generator is configured to radiate the signal light having a predetermined beam diameter to the cornea and generate the interference light having a predetermined beam diameter;
the detector is configured to detect the interference light on a two-dimensional light-receiving face; and
the forming part is configured to form a two-dimensional image of a region of the cornea corresponding to the beam diameter of the signal light, as the image.

27. The cornea observation device according to Claim 25, wherein:

the interference-light generator is configured to radiate the signal light having a predetermined beam diameter to the cornea and generate the interference light having a predetermined beam diameter;
the detector is configured to detect the interference light on a two-dimensional light-receiving face; and
the forming part is configured to form a two-dimensional image of a region of the cornea corresponding to the beam diameter of the signal light, as the image.

28. The cornea observation device according to Claim 24, wherein the image forming part includes a changing part configured to change a difference in optical path length between the signal light and the reference light, and is configured to form an image of the cornea at a depth position corresponding to the difference in optical path length.

29. The cornea observation device according to Claim 25, wherein the image forming part includes a changing part configured to change a difference in optical path length between the signal light and the reference light, and is configured to form an image of the cornea at a depth position corresponding to the difference in optical path length.

# FIG. 1

# FIG. 2

# FIG. 3

```
                    ┌─────────────┐
                    │    START    │
                    └─────────────┘
                           │
          ┌────────────────────────────────┐
          │      EXECUTE ALIGNMENT          │── S1
          └────────────────────────────────┘
                           │
          ┌────────────────────────────────┐
          │  FORM TOMOGRAPHIC IMAGE IN      │── S2
          │  HORIZONTAL DIRECTION OF CORNEA │
          └────────────────────────────────┘
                           │
          ┌────────────────────────────────┐
          │  EXTRACT CELLULAR IMAGE REGION  │── S3
          │  FROM TOMOGRAPHIC IMAGE         │
          └────────────────────────────────┘
                           │
          ┌────────────────────────────────┐
          │  GENERATE CELLULAR INFORMATION  │── S4
          └────────────────────────────────┘
                           │
          ┌────────────────────────────────┐
          │  SPECIFY DEPTH POSITION OF      │── S5
          │  IMAGE IN CORNEA                │
          └────────────────────────────────┘
                           │
          ┌────────────────────────────────┐
          │  DISPLAY RESULT OF SPECIFICATION│── S6
          │  OF DEPTH POSITION              │
          └────────────────────────────────┘
                           │
          ┌────────────────────────────────┐
          │  DETERMINE WHETHER THERE IS     │── S7
          │  ABNORMALITY OF CELL            │
          └────────────────────────────────┘
                           │
          ┌────────────────────────────────┐
          │  DISPLAY RESULT OF DETERMINATION│── S8
          │  WHETHER THERE IS ABNORMALITY   │
          └────────────────────────────────┘
                           │
                    ┌─────────────┐
                    │     END     │
                    └─────────────┘
```

## INTERNATIONAL SEARCH REPORT

| International application No. |
|---|
| PCT/JP2008/001511 |

A. CLASSIFICATION OF SUBJECT MATTER
*A61B3/12*(2006.01)i

According to International Patent Classification (IPC) or to both national classification and IPC

B. FIELDS SEARCHED

Minimum documentation searched (classification system followed by classification symbols)
A61B3/12

Documentation searched other than minimum documentation to the extent that such documents are included in the fields searched
Jitsuyo Shinan Koho        1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 6-274600 A  (Konan Inc.),<br>30 September, 1994 (30.09.94),<br>& US 5523213 A          & EP 621477 A3 | 1-22 |
| A | Takashi MIYAI, Kazunori MIYATA, "Q5 Specular Microscope no Yomikata ni Tsuite Oshiete Kudasai", Journal of the Eye, 30 March, 2007 (30.03.07), special extra issue, whole No.295, pages 126 to 128, Okuzuke | 1-22 |

☐ Further documents are listed in the continuation of Box C.   ☐ See patent family annex.

| | |
|---|---|
| *    Special categories of cited documents:<br>"A"  document defining the general state of the art which is not considered   to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>14 August, 2008 (14.08.08) | Date of mailing of the international search report<br>26 August, 2008 (26.08.08) |
|---|---|
| Name and mailing address of the ISA/<br>Japanese Patent Office | Authorized officer |
| Facsimile No. | Telephone No. |

Form PCT/ISA/210 (second sheet) (April 2007)

## INTERNATIONAL SEARCH REPORT

| International application No. |
| --- |
| PCT/JP2008/001511 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
A specific matter common to claims 1-22 (hereinafter called the main invention) and claims 23-29 (hereinafter called the second invention) is a matter that "irradiates light to the eye under examination, detects the light by way of the cornea, includes an image forming means for forming a cell image of the cornea based on this detected result, analyzes a form of the cell displayed on the image, but the specific matter has been well known as a specular microscope as described in the following:
Document 1: JP 6-274600 A (Konan Inc.), 30 September, 1994 (30.09.94), & US 5523213 A & EP 621477 A, or
                                        (To be continued on the extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.    Consequently, this international search report is restricted to the invention first mentioned in the claims; it is covered by claims Nos.: 1-22 .

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| **INTERNATIONAL SEARCH REPORT** | International application No. |
|---|---|
| | PCT/JP2008/001511 |

<u>Continuation of Box No.III of continuation of first sheet(2)</u>

Document 2: Takashi MIYAI, Kazunori MIYATA, "Q5 Specular Microscope no Yomikata ni Tsuite Oshiete Kudasai", Journal of the Eye, 30 March, 2007 (30.03.07), special extra issue, whole No.295, pages 126-128, colophon.

Since the common matter does not make any contribution over the prior art, it is not the special technical feature in the meaning of PCT Rule 13.2, second sentence.

Thus, as far as the main invention is compared with the prior art set forth above, a technical feature of the main invention is directed to "a specifying means for specifying a depth position of the cornea shown in an image".

Further, as far as the second invention is compared with the prior art set forth above, a technical feature of the second invention is directed to "an analyzing means for judging if a cornea cell has an abnormality".

The technical features of both inventions are completely different from each other, and no technical relationship to involve one or more of the same or corresponding special technical features is deemed to exist between them.

Therefore, this international application includes two inventions that do not comply with the requirement of unity of invention.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9098950 A
- JP 10090606 A
- JP 9084763 A
- JP 2006153838 A
- JP 2006116028 A
- JP 7092656 A